# FASCICULE DE BREVET EUROPEEN

(11) **EP 4 161 285 B1**
(45) Date de publication et mention de la délivrance du brevet: **24.07.2024**
(21) Numéro de dépôt: 21737706.8
(22) Date de dépôt: 04.06.2021
(51) Int. Cl.: A23K 20/174, A23L 33/105, A23L 33/155, A61K 36/18, A61P 39/06, C11B 5/00

(54) **FORMULATION DE VITAMINE A**
VITAMIN-A-FORMULIERUNG
VITAMIN A FORMULATION

(30) Priorité: 05.06.2020 FR 2005883
(43) Date de publication de la demande: 12.04.2023
(73) Titulaire: Adisseo France S.A.S., 92160 Antony (FR)
(72) Inventeur: ALEMAN, Claude, 03600 COMMENTRY (FR); HUET, Robert, 75015 PARIS (FR)
(74) Mandataire: Germain Maureau
(86) Numéro de dépôt international: PCT/FR2021/051011
(87) Numéro de publication internationale: WO 2021/245362

(56) Documents cités:
- WO-A1-2013/036934
- WO-A1-2015/059270
- WO-A1-2016/189345
- FR-A1- 2 899 768
- FR-B1- 2 899 768
- GB-A- 2 184 341
- US-A- 5 498 434

## Description

L'invention concerne l'utilisation d'un mélange d'acide carnosique et de carnosol comme agent antioxydant dans des formulations de principes actifs hydrophobes et liposolubles.

Ces molécules, comme les vitamines, les acides gras, sont très largement employées dans de nombreux domaines techniques tels que les industries pharmaceutique, cosmétique, agroalimentaire, et notamment dans le domaine de la nutrition animale. A titre d'exemple, les vitamines liposolubles A, E et D3 sont couramment utilisées pour la préparation d'aliments favorisant la croissance d'animaux, et notamment les animaux d'élevage.

Leur nature hydrophobe et leur fragilité environnementale, notamment thermique et chimique, tant au cours de leur formulation et de leur stockage, que lors de leur utilisation, rendent nécessaire leur préservation par l'intégration d'agents protecteurs et/ou par une fabrication adaptée, notamment par encapsulation.

La présente invention est ci-après plus particulièrement décrite en référence à la vitamine A, éventuellement associée à une ou d'autres vitamines liposolubles telles que les vitamines E et D3 mais, bien entendu, son cadre n'y est pas restreint, et elle peut s'appliquer à toute substance active hydrophobe et liposoluble et tout mélange de telles substances.

La vitamine A existe sous forme libre mais aussi sous d'autres formes, notamment à l'état d'ester, les formes les plus stables et donc les plus couramment utilisées étant l'acétate de vitamine A, le propionate de vitamine A et le palmitate de vitamine A. Dans le présent texte, on inclura dans le terme vitamine A, l'une quelconque de ces formes, notamment libre ou estérifiée, ainsi que tout mélange d'au moins deux quelconques de ces formes.

Même sous ses formes les plus stables, la vitamine A reste très sensible à l'oxydation résultant d'agents oxydants tels que l'oxygène, la température, l'eau et la lumière, ainsi qu'à l'action des acides. En application pharmaceutique ou en nutrition animale, elle est ainsi très rapidement dégradée dès qu'elle entre en contact avec les premières conditions sévères, notamment acides, du système digestif, ce qui n'en fait pas une forme biodisponible de la vitamine A.

Afin de préserver au mieux la vitamine A, il est connu depuis longtemps de la protéger vis-à-vis de l'oxydation en l'associant à un antioxydant. Un tel agent est généralement choisi parmi le 3-tert-butyl-4-hydroxy-anisole (BHA), le 2,6-di-tert-butyl-4-méthyl-phénol (BHT), le 6-éthoxy-1,2-dihydroxy-2,2,4-triméthyl-quinoléine (éthoxyquinine), le 2-tertiobutyl-1,4-dihydroxy-benzène ou encore le tocophérol, l'acide citrique ou l'acide phytique et leurs sels de métaux alcalins, ou encore l'acide éthylène diamine tetra acétique (EDTA) et l'acide ascorbique. Parmi ceux-ci, le BHT reste le plus employé en raison de son efficacité puissante pour un faible coût et sa stabilité à la température. Il n'est cependant pas dénué de toxicité et est de plus en plus décrié, suspecté d'être un perturbateur endocrinien, ainsi que pouvant être responsable d'allergies et de favoriser le développement de tumeurs existantes.

Ainsi, l'invention vise à remplacer au moins en partie, voire en totalité, les antioxydants issus de synthèse chimique classiquement utilisés et en particulier le BHT, par un antioxydant plus naturel au sens où il peut être obtenu par des voies recourant à moins de chimie, peu, voire non nocif et facilement accessible.

Des recherches ont déjà été engagées dans ce sens, notamment pour l'alimentation humaine. Ainsi, certaines plantes de la famille des Lamiaceae connues pour avoir un effet antioxydant, ont fait l'objet de travaux et notamment le romarin (dont le nom scientifique est *Rosmarinus officinalis).*

Selon le document R. Inatani et al. Agric. Biol. Chem. (1983) 47 (3), 521, l'activité antioxydante de différents extraits de feuilles de romarin est déterminée. Ces extraits sont obtenus en utilisant différents solvants, n-hexane, dichlorométhane et éthanol et leur activité antioxydante est mesurée. A partir des extraits manifestant une forte activité antioxydante, des fractions sont séparées, dont l'activité antioxydante est mesurée. Des composés sont isolés des fractions ayant la plus forte activité parmi lesquels, le rosmanol et le carnosol possèdent le pouvoir antioxydant le plus élevé.

Le document N. Nakatani et al. Agric. Biol. Chem. (1984) 48 (8), 2081 se rapporte à l'isolement de deux isomères du rosmanol, l'épirosmanol et l'isorosmanol, à partir d'un extrait de romarin.

Le document M. Loussouarn et al. Plant Physiology (2017) 175, 1381 concerne une étude sur les mécanismes d'action de l'acide carnosique et du carnosol en tant qu'antioxydant vis-à-vis des lipides membranaires, *in vitro* et *in vivo.*

Ces travaux mentionnent que, selon la méthode de mesure du pouvoir antioxydant et les concentrations testées, certains des composés isolés ont une activité se rapprochant de celle du BHT ou du BHA, qui peut même être similaire, voire supérieure.

Selon le document CN109452467, il a été décrit des microcapsules de la vitamine A, dont le coeur comporte la vitamine A en une teneur pouvant atteindre 54% en poids par rapport au poids de la microcapsule, en mélange avec du palmitate d'ascorbyle, de l'acide carnosique et de la choline ou dérivé et éventuellement une huile, et l'enveloppe est à base de gélatine et de polysaccharide(s). Les auteurs ont procédé à des tests de vieillissement comparatifs avec différentes microcapsules ne répondant pas à la définition ci-dessus et notamment des microcapsules dont le coeur ne comprend pas de choline ou dérivé, et des microcapsules dont le coeur ne comprend ni palmitate d'ascorbyle, ni choline ou dérivé. Les résultats démontrent que la présence de l'association palmitate d'ascorbyle, acide carnosique et choline ou dérivé est indispensable bien que non suffisante pour conférer à la formulation de vitamine A une stabilité suffisante pour une utilisation en alimentation ou nutrition animale.

Le document WO 2016/189345 A1 décrit une composition à visée notamment thérapeutique, comprenant deux huiles végétales présentes chacune en une teneur de 35-50% (p/p), au moins un phytostérol, 0,005-0,15% (p/p) d'au moins la vitamine A ou la vitamine D et 0,02-0,2% (p/p) d'un antioxydant qui peut être un extrait de romarin et aussi un mélange d'un extrait de romarin, de tocophérol et/ou de palmitate d'ascorbyle.

L'invention apporte une solution permettant de surmonter l'ensemble des problèmes rencontrés par les formulations existantes en fournissant un antioxydant qui présente vis-à-vis de la vitamine A et des vitamines liposolubles qui lui sont éventuellement associées, un effet protecteur à l'égard de l'oxydation qui se révèle très supérieur à celui du BHT ou du BHA. Il est au surplus d'origine naturelle. Selon l'invention, cet antioxydant comprend ou consiste en un mélange d'acide carnosique et de carnosol, la teneur en acide carnosique dudit agent antioxydant étant de 50-98% en poids par rapport au poids dudit agent antioxydant.

Comme il sera démontré dans les exemples, le pouvoir antioxydant de ces extraits est similaire à celui du BHT ou du BHA dans des proportions pourtant inférieures.

Ainsi, un objet de l'invention est une association d'au moins la vitamine A et d'un agent antioxydant comprenant ou consistant en un mélange d'acide carnosique et de carnosol, la teneur en acide carnosique dudit agent antioxydant étant de 50-98% en poids par rapport au poids dudit agent antioxydant.

Selon une mise en oeuvre de l'invention, la teneur en carnosol dudit agent antioxydant est de 2-15% en poids par rapport au poids dudit agent antioxydant.

L'agent antioxydant de l'invention peut consister en ledit mélange d'acide carnosique et de carnosol et ainsi se substituer totalement à tous autres agents antioxydants classiquement employés. Dans cette variante, il a été observé qu'il peut être efficace en une concentration par rapport à la vitamine A très inférieure à celles des antioxydants usuels comme le BHT. Il a été constaté de manière surprenante que certains véhicules potentialisent son effet antioxydant, c'est le cas de certaines huiles, des huiles de préférence comestibles comme l'huile de tournesol. Le mélange d'acide carnosique et carnosol peut donc se présenter sous forme de poudre ou bien dans un véhicule tel qu'une huile comestible.

L'agent antioxydant de l'invention peut aussi comprendre ledit mélange d'acide carnosique et de carnosol. Ainsi, en plus de ce mélange, il peut comprendre tout autre agent antioxydant de la liste citée précédemment, comme le BHT, le BHA, l'EDTA et l'acide ascorbique, ou tout composé se révélant antioxydant à l'égard de la vitamine A. Il a en effet été observé que l'incorporation de composés naturels tels que des polyphénols, ou d'extraits naturels, notamment riches en polyphénols, et en particulier d'un extrait de thé vert, d'un extrait de pépins de raisin, ou un mélange de ceux-ci agit favorablement sur le pouvoir antioxydant dudit mélange.

Une association peut comprendre la vitamine A comme seul ingrédient actif liposoluble, mais elle peut aussi comprendre en plus de la vitamine A, une ou d'autres vitamines liposolubles comme la vitamine E et la vitamine D3.

Comme indiqué précédemment, l'antioxydant est accessible à partir de sources végétales. Le mélange d'acide carnosique et de carnosol précité peut être effectivement présent sous la forme d'un extrait de Lamiaceae et de préférence un extrait de romarin, de sauge ou de menthe.

Par extrait de romarin, de sauge ou de menthe, on entend un extrait obtenu par au moins une étape d'extraction ; on comprend aussi que l'extrait a subi un ou des traitements complémentaires entraînant la concentration dudit extrait en certains composés et notamment en acide carnosique et/ou en carnosol. Les extraits de romarin, sauge ou menthe peuvent être des extraits alcooliques ou hydroalcooliques ou des extraits de CO₂ surpercritique de feuilles de romarin, sauge ou menthe, selon des techniques classiques bien connues de l'homme du métier. Des extraits répondant aux caractéristiques de l'invention sont aussi commercialisés. Avantageusement selon l'invention, on recourt à des extraits de romarin.

L'extrait de Lamiaceae peut se présenter sous forme de poudre ou dans un véhicule tel qu'une huile comestible.

Selon une mise en oeuvre de l'invention, la teneur en vitamine A, et optionnellement en au moins l'une de la vitamine E et la vitamine D3, dans l'association est de 75-99% en poids par rapport au poids de l'association.

L'invention concerne aussi une formulation de vitamine A comprenant au moins une association telle que définie précédemment, un sucre et un agent gélifiant.

Pour une mise en oeuvre bénéfique de l'invention, une telle formulation comprend de préférence 30-35% en poids de vitamine A et optionnellement d'au moins l'une de la vitamine E et de la vitamine D3, 1-10% en poids dudit agent antioxydant, 25-30% en poids d'un sucre et 23-28% en poids d'un agent gélifiant, les pourcentages étant exprimés en poids par rapport au poids de la formulation.

Une formulation de l'invention peut comprendre un ou des additifs choisis parmi des huiles comestibles, des agents de réticulation, des agents-anti-agglomérants, des tensio-actifs.

Une formulation de l'invention peut se présenter sous forme de poudre, par exemple dont la taille des particules est de préférence de l'ordre de 250 à 350 µm, sous forme de microbilles.

Il a été observé de manière inattendue, qu'en plus de son pouvoir antioxydant, le mélange d'acide carnosique et de carnosol a un pouvoir chélatant susceptible alors de renforcer la protection de la vitamine A vis-à-vis de cations délétères qui seraient présents dans des ingrédients de la formulation. C'est un avantage supplémentaire par rapport aux antioxydants classiques et notamment le BHT.

Comme il sera présenté plus en détails ci-après, une formulation de l'invention peut être obtenue par double émulsion ou par atomisation d'une émulsion ; une telle émulsion comprend de préférence au moins 10 à 22% en poids de vitamine A et optionnellement au moins l'une de la vitamine E et de la vitamine D3, 0,5 à 5% en poids d'un agent antioxydant de l'invention, 13 à 20% en poids d'undit sucre et 12 à 18% en poids d'undit agent gélifiant.

Les formulations de vitamine A de l'invention peuvent être préparées par des procédés connus tels que ceux décrits dans EP285682A ou WO2011/070300A.

Comme détaillé dans le document EP285682A, on prépare une émulsion contenant la vitamine A, de l'eau, un antioxydant, de la gélatine et un sucre que l'on transforme en gouttelettes par pulvérisation. Ces gouttelettes sont ensuite mises en contact individuellement avec une poudre de cellulose présentant des caractéristiques bien définies jusqu'au durcissement des gouttelettes. Les gouttelettes ainsi durcies sont alors séparées de la poudre de cellulose par tamisage. Les gouttelettes récupérées sont ensuite séchées, puis soumises à une opération de chauffage afin d'assurer la réticulation de la gélatine par réaction des groupes aminés de la gélatine avec les fonctions réductrices du sucre.

On peut aussi fabriquer une formulation selon le procédé décrit par le document WO2011/070300A selon les étapes suivantes :
on prépare une émulsion huile dans l'eau comprenant en pourcentage en poids par rapport au poids total de ladite émulsion :
   8 à 20 % d'au moins une protéine, préférentiellement 10 à 15%,
   5 à 15 % d'au moins un sucre, préférentiellement 8 à 12%,
   0,5 à 3 % d'au moins un sel inorganique, préférentiellement 2 à 3%,
   0,68-5% d'un antioxydant de l'invention,
   10 à 22% d'au moins un principe actif liposoluble et en particulier la vitamine A sous forme huileuse et/ou dissous dans une huile alimentaire, préférentiellement 15 à 20%,
qsp % d'eau,
on procède au formage de particules sous la forme substantiellement sphériques par la dispersion de l'émulsion huile dans l'eau obtenue à l'issue de l'étape a) dans un fluide,
on ajoute au moins un agent de réticulation de la protéine à la dispersion,
on récupère les principes actifs sous la forme substantiellement sphérique.

Selon des mises en oeuvre particulières de ce procédé :
la ou les protéines sont choisies parmi la gélatine, la caséine ou la caséinate, les protéines du soja ou du maïs ;
le ou les sucres sont choisis parmi les polyols, les monosaccharides, les disaccharides, les sirops de glucose et de fructose et les maltodextrines ;
le ou les sels inorganiques sont choisis parmi les sels de sodium, potassium, lithium d'acide mono-, di- et tri- phosphorique ou d'acide polyphosphorique, de préférence le Na₂HPO₄.

Ce procédé de préparation peut aussi comprendre une étape préalable lors de laquelle on dissout la vitamine A dans une huile alimentaire telle que l'huile d'arachide, de tournesol, de colza, de maïs, de soja, de palme ou de foie de morue.

L'invention concerne aussi un aliment ou fourrage pour la nutrition animale comprenant une formulation de vitamine A telle que décrite ci-dessus dans l'une quelconque de ses variantes.

L'invention se rapporte en outre à une utilisation d'un mélange d'acide carnosique et de carnosol comme ou dans un agent antioxydant dans une formulation de vitamine A, et optionnellement d'au moins l'une de la vitamine E et de la vitamine D3. De préférence, la teneur en acide carnosique dudit agent antioxydant est de 50-98%, et de manière encore préférée, la teneur en carnosol dudit agent antioxydant est de 2-15% en poids par rapport au poids dudit agent antioxydant.

L'invention est ci-après illustrée dans les exemples suivants, décrivant en détails certaines mises en oeuvre et démontrant ses performances.

### Exemples :

Les méthodes de mesure du pouvoir antioxydant utilisées dans les exemples pour des mélanges antioxydant et vitamine A (sous la forme d'acétate de vitamine A) sont indiquées ci-dessous.

### 1) Test d'oxydation dynamique DSC (Differencial Scanning Calorimeter).

Il est réalisé sous flux d'oxygène (50 ml/min) puis remontée en température à une température supérieure au point de fusion de la vitamine A (soit supérieure à 60°C).

L'oxydation correspond à un phénomène exothermique qui intervient à une température donnée. Pour le BHT, il se produit à 153°C. Plus la température d'oxydation est haute plus le pouvoir antioxydant est élevé.

Tous les échantillons présentant une température supérieure ou égale à 120°C seront retenus pour un test Oxipres beaucoup plus long.

### 2) Test Oxipres :

Ce test consiste à mettre les échantillons sous pression d'Oz à une température donnée. La méthode permet de déterminer la cinétique de consommation d'oxygène, exprimée par une variation d'Oz (ΔP en bars), cette variation étant d'autant plus faible que le pouvoir antioxydant est élevé.

### Exemple 1 : Préparation des échantillons à tester

Les échantillons 2-6 sont préparés en mélangeant de l'acétate de vitamine A et un antioxydant présent dans l'association en une teneur de 20% en poids.

Ils sont ensuite testés selon les modèles d'évaluation décrits ci-dessus, ainsi qu'un échantillon 1 d'acétate de vitamine A (sans antioxydant).

### Exemple 2 : Résultats des tests DSC 1) et Oxipres sur mélange binaires

Les résultats des tests sur les échantillons 1-6 sont présentés dans le tableau suivant. Le mélange acide carnosique/carnosol est abrégé AC/c.

**[Tableau 1]**

| Echantillon N° | Antioxydant | DSC DYN Onset (°C) | Oxipres Temps (h) Δ P (Bars) |
|---|---|---|---|
| 1 | Aucun | 60°C | 0 |
| | | | 5,0 |
| 2 | BHT | 153,60 | >168 |
| | | | Δ P = 1,8 |
| 3 | α Tocophérol | 127,00 | >168 |
| | | | Δ P = 2,25 |
| 4 | AC/c +extrait de thé vert et extrait de pépin de raisin* | 130,00 | >168 |
| | | | Δ P = 1,2 |
| 5 | AC 85%/c 15% sous forme d'extrait de romarin | 132,00 | >168 |
| | | | Δ P = 1,2 |
| 6 | AC 85%/c 15% dans de l'huile de tournesol 4%) | 143,00 | |

| | | | |
|---|---|---|---|
| * le mélange AC/c + extrait de thé vert et extrait de pépin de raisin est commercialisé par LAYN sous la référence TruGro^{®}11136-19080504. | | | |

On observe que le pouvoir des antioxydants des échantillons 4-6 illustrant l'invention est toujours supérieur à celui de l'α-tocophérol dans les deux tests d'évaluation, et se rapproche, voire est supérieur dans le test d'évaluation Oxipres, à celui du BHT. On constate en outre que ce pouvoir s'exprime à des teneurs très faibles du mélange AC/c, le résultat de l'échantillon 6 est particulièrement intéressant.

## Revendications

1. Association d'au moins la vitamine A et d'un agent antioxydant comprenant ou consistant en un mélange d'acide carnosique et de carnosol, la teneur en acide carnosique dudit agent antioxydant étant de 50-98% en poids par rapport au poids dudit agent antioxydant.

2. Association selon la revendication 1, **caractérisée en ce que** la teneur en carnosol dudit agent antioxydant est de 2-15% en poids par rapport au poids dudit agent antioxydant.

3. Association selon la revendication 1 ou 2, **caractérisée en ce que** l'antioxydant comprend en outre au moins l'un du BHT, du BHA, de l'EDTA et de l'acide ascorbique.

4. Association selon l'une quelconque des revendications 1 à 3, **caractérisée en ce que** l'antioxydant comprend en outre des polyphénols, tels que des polyphénols naturels et notamment un extrait de thé vert ou un extrait de pépins de raisin ou un mélange desdits extraits.

5. Association selon l'une quelconque des revendications 1 à 4, de la vitamine A et d'au moins une vitamine choisie parmi la vitamine E et la vitamine D3.

6. Association selon l'une quelconque des revendications 1 à 5, **caractérisée en ce que** le mélange d'acide carnosique et de carnosol est présent sous la forme d'un extrait de Lamiaceae et de préférence un extrait de romarin, de sauge ou de menthe.

7. Association selon la revendication 6, **caractérisée en ce que** l'extrait de romarin est choisi parmi des extraits alcooliques ou hydroalcooliques et des extraits de CO₂ surpercritique de feuilles de romarin.

8. Association selon l'une quelconque des revendications 1 à 7, **caractérisée en ce que** ledit mélange ou ledit extrait de Lamiaceae est sous forme de poudre ou dans un véhicule tel qu'une huile comestible.

9. Association selon l'une quelconque des revendications 1 à 8, **caractérisée en ce que** la teneur en vitamine A, et optionnellement en au moins l'une de la vitamine E et la vitamine D3, est de 75-99% en poids par rapport au poids de l'association.

10. Formulation d'au moins la vitamine A comprenant au moins une association selon l'une quelconque des revendications 1 à 9, un sucre et un agent gélifiant.

11. Formulation selon la revendication 10, **caractérisée en ce qu'**elle comprend 30-35% en poids de vitamine A et optionnellement d'au moins l'une de la vitamine E et de la vitamine D3, 1-10% en poids dudit agent antioxydant, 25-30% en poids d'un sucre et 23-28% en poids d'un agent gélifiant.

12. Formulation selon la revendication 10 ou 11, **caractérisée en ce qu'**elle comprend un ou des additifs choisis parmi des huiles comestibles, des agents de réticulation, des agents-anti-agglomérants, des tensio-actifs.

13. Formulation selon l'une quelconque des revendications 10 à 12, **caractérisée en ce qu'**elle est obtenue par double émulsion ou atomisation d'une émulsion comprenant au moins 10-22% en poids de vitamine A et optionnellement au moins l'une de la vitamine E et de la vitamine D3, 0,5-5% en poids dudit agent antioxydant, 13-20% en poids dudit sucre et 12-18% en poids dudit agent gélifiant.

14. Formulation selon l'une quelconque des revendications 10 à 13, se présentant sous la forme de poudre.

15. Aliment ou fourrage pour la nutrition animale comprenant une formulation de vitamine A selon l'une quelconque des revendications 10 à 14.

16. Utilisation d'un mélange d'acide carnosique et de carnosol comme ou dans un agent antioxydant dans une formulation de vitamine A, et optionnellement d'au moins l'une de la vitamine E et de la vitamine D3.

## Patentansprüche

1. Zusammensetzung aus mindestens Vitamin A und einem antioxidativen Mittel, das eine Mischung aus Carnosinsäure und Carnosol umfasst oder daraus besteht, wobei der Carnosinsäuregehalt des antioxidativen Mittels 50-98 Gew.-%, bezogen auf das Gewicht des antioxidativen Mittels, beträgt.

2. Zusammensetzung nach Anspruch 1, **dadurch gekennzeichnet, dass** der Carnosolgehalt des antioxidativen Mittels 2-15 Gew.-%, bezogen auf das Gewicht des antioxidativen Mittels, beträgt.

3. Zusammensetzung nach Anspruch 1 oder 2, **dadurch gekennzeichnet, dass** das Antioxidans zusätzlich mindestens eines von BHT, BHA, EDTA und Ascorbinsäure umfasst.

4. Zusammensetzung nach einem der Ansprüche 1 bis 3, **dadurch gekennzeichnet, dass** das Antioxidans zusätzlich Polyphenole, wie beispielsweise natürliche Polyphenole und insbesondere einen Grüntee- oder Traubenkernextrakt oder eine Mischung der Extrakte umfasst.

5. Zusammensetzung nach einem der Ansprüche 1 bis 4, die aus Vitamin A und mindestens einem Vitamin, ausgewählt aus Vitamin E und Vitamin D3, besteht.

6. Zusammensetzung nach einem der Ansprüche 1 bis 5, **dadurch gekennzeichnet, dass** die Mischung aus Carnosinsäure und Carnosol in Form eines Lamiaceae-Extrakts und vorzugsweise eines Rosmarin-, Salbei- oder Minzextrakts vorliegt.

7. Zusammensetzung nach Anspruch 6, **dadurch gekennzeichnet, dass** der Rosmarinextrakt aus alkoholischen oder wässrig-alkoholischen Extrakten und überkritischen CO₂-Extrakten von Rosmarinblättern ausgewählt ist.

8. Zusammensetzung nach einem der Ansprüche 1 bis 7, **dadurch gekennzeichnet, dass** die Mischung oder der Lamiaceae-Extrakt in Form eines Pulvers oder in einem Vehikel wie einem Speiseöl vorliegt.

9. Zusammensetzung nach einem der Ansprüche 1 bis 8, **dadurch gekennzeichnet, dass** der Gehalt an Vitamin A und optional mindestens eines der Vitamine E und D3 75-99 Gew.-%, bezogen auf das Gewicht der Zusammensetzung, beträgt.

10. Formulierung von mindestens Vitamin A, umfassend mindestens eine Kombination nach einem der Ansprüche 1 bis 9, einen Zucker und ein Geliermittel.

11. Formulierung nach Anspruch 10, **dadurch gekennzeichnet, dass** sie 30-35 Gew.-% Vitamin A und optional mindestens eines von Vitamin E und Vitamin D3, 1-10 Gew.-% des antioxidativen Mittels, 25-30 Gew.-% eines Zuckers und 23-28 Gew.-% eines Geliermittels umfasst.

12. Formulierung nach Anspruch 10 oder 11, **dadurch gekennzeichnet, dass** sie einen oder mehrere Zusatzstoffe umfasst, die aus Speiseölen, Vernetzungsmitteln, Trennmitteln und Tensiden ausgewählt sind.

13. Formulierung nach einem der Ansprüche 10 bis 12, **dadurch gekennzeichnet, dass** sie durch Doppelemulsion oder Zerstäubung einer Emulsion erhalten wird, die mindestens 10-22 Gew.-% Vitamin A und optional mindestens eines der Vitamine E und D3, 0,5-5 Gew.-% des antioxidativen Mittels, 13-20 Gew.-% des Zuckers und 12-18 Gew.-% des Geliermittels umfasst.

14. Formulierung nach einem der Ansprüche 10 bis 13, die in Pulverform vorliegt.

15. Nahrungs- oder Futtermittel für Lebewesen, umfassend eine Vitamin-A-Formulierung nach einem der Ansprüche 10 bis 14.

16. Verwendung einer Mischung aus Carnosinsäure und Carnosol als oder in einem antioxidativen Mittel in einer Formulierung von Vitamin A, und optional mindestens eines von Vitamin E und Vitamin D3.

## Claims

1. A combination of at least vitamin A and an antioxidant agent comprising or consisting of a mixture of carnosic acid and carnosol, the carnosic acid content of said antioxidant agent being 50-98% by weight relative to the weight of said antioxidant agent.

2. The combination according to claim 1, **characterized in that** the carnosol content of said antioxidant agent is 2-15% by weight relative to the weight of said antioxidant agent.

3. The combination according to claim 1 or 2, **characterized in that** the antioxidant further comprises at least one of BHT, BHA, EDTA and the ascorbic acid.

4. The combination according to any one of claims 1 to 3, **characterized in that** the antioxidant further comprises polyphenols, such as natural polyphenols and in particular a green tea extract or a grape seed extract or a mixture of said extracts.

5. The combination according to any one of claims 1 to 4, of vitamin A and at least one vitamin selected from vitamin E and vitamin D3.

6. The combination according to any one of claims 1 to 5, **characterized in that** the mixture of carnosic acid and carnosol is present in the form of Lamiaceae extract and preferably a rosemary, sage or mint extract.

7. The combination according to claim 6, **characterized in that** the rosemary extract is selected from alcoholic or hydroalcoholic extracts and supercritical CO₂ extracts of rosemary leaves.

8. The combination according to any one of claims 1 to 7, **characterized in that** said mixture or said Lamiaceae extract is in powder form or is present in a vehicle such as an edible oil.

9. The combination according to any one of claims 1 to 8, **characterized in that** the vitamin A content, and optionally at least one of vitamin E and vitamin D3 contents, is 75-99% by weight relative to the weight of the combination.

10. A formulation of at least vitamin A comprising at least one combination according to any one of claims 1 to 9, a sugar and a gelling agent.

11. The formulation according to claim 10, **characterized in that** it comprises 30-35% by weight of vitamin A and optionally at least one of vitamin E and vitamin D3, 1-10% by weight of said antioxidant agent, 25-30% by weight of a sugar and 23-28% by weight of a gelling agent.

12. The formulation according to claim 10 or 11, **characterized in that** it comprises one or more additive(s) selected from edible oils, crosslinking agents, anti-caking agents, surfactants.

13. The formulation according to any one of claims 10 to 12, **characterized in that** it is obtained by double emulsion or by atomization of an emulsion comprising at least 10-22% by weight of vitamin A and optionally at least one of vitamin E and vitamin D3, 0.5-5% by weight of said antioxidant agent, 13-20% by weight of said sugar and 12-18% by weight of said gelling agent.

14. The formulation according to any one of claims 10 to 13, which is in the powder form.

15. A feed or fodder for animal nutrition comprising a vitamin A formulation according to any one of claims 10 to 14.

16. A use of a mixture of carnosic acid and carnosol as or in an antioxidant agent in a vitamin A formulation, and optionally at least one of vitamin E and vitamin D3.
